# EUROPEAN PATENT SPECIFICATION

(11) **EP 0 522 527 B1**
(45) Date of publication and mention of the grant of the patent: **21.12.1994**
(21) Application number: 92111579.6
(22) Date of filing: 08.07.1992
(51) Int. Cl.: A61M 5/142

(54) **Solution infusion system**
Flüssigkeitslösung-Infusionssystem
Système de perfusion des solutions liquides

(30) Priority: 10.07.1991 JP 169729/91
(43) Date of publication of application: 13.01.1993
(73) Proprietor: SHARP KABUSHIKI KAISHA, Osaka (JP)
(72) Inventor: Hara, Keita, Kashihara-shi, Nara-ken (JP)
(74) Representative: Müller, Frithjof E., Dipl.-Ing.

(56) References cited:
- EP-A- 0 238 809
- EP-A- 0 346 548
- CH-A- 604 741
- US-A- 4 174 637

## Description

### BACKGROUND OF THE INVENTION

### Field of the Invention

The present invention relates to a solution infusion system for infusing a solution into a patient.

### Description of the Prior Art

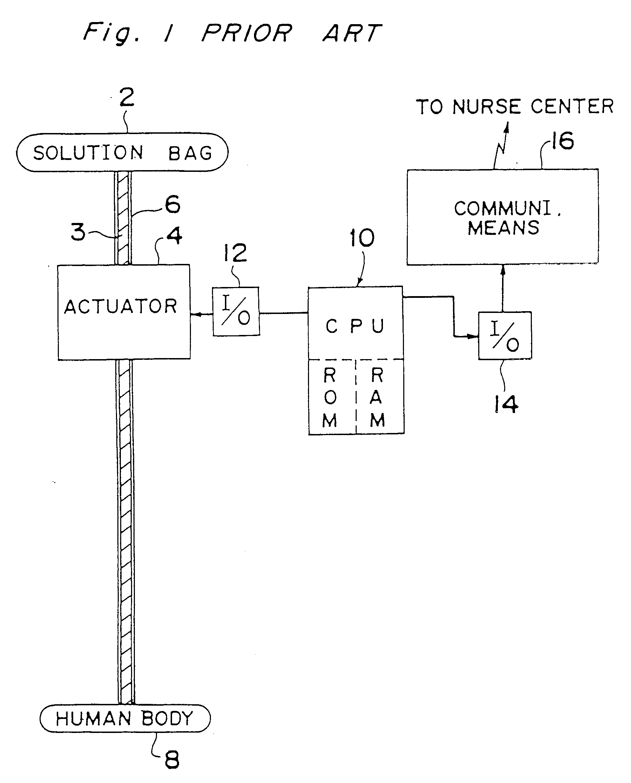

Fig. 1 depicts a conventional solution infusion system wherein a solution 3 contained in a solution bag 2 is introduced into a tube 6 by the pumping action of a solution actuator 4 and is injected into a human body 8 via an injector needle (not shown). A control unit 10 is provided with a microcomputer, which comprises a CPU for executing a main control operation, a ROM in which is stored an infusion operation control program, and a RAM for temporarily storing data. The control unit 10 controls the solution actuator 4 via an I/O interface 12 in accordance with the infusion operation control program, thereby feeding an appropriate amount of solution to the human body 8. Also, the control unit 10 informs a nurse center or the like of the solution conditions via an I/O interface 14 and any suitable communication means 16.

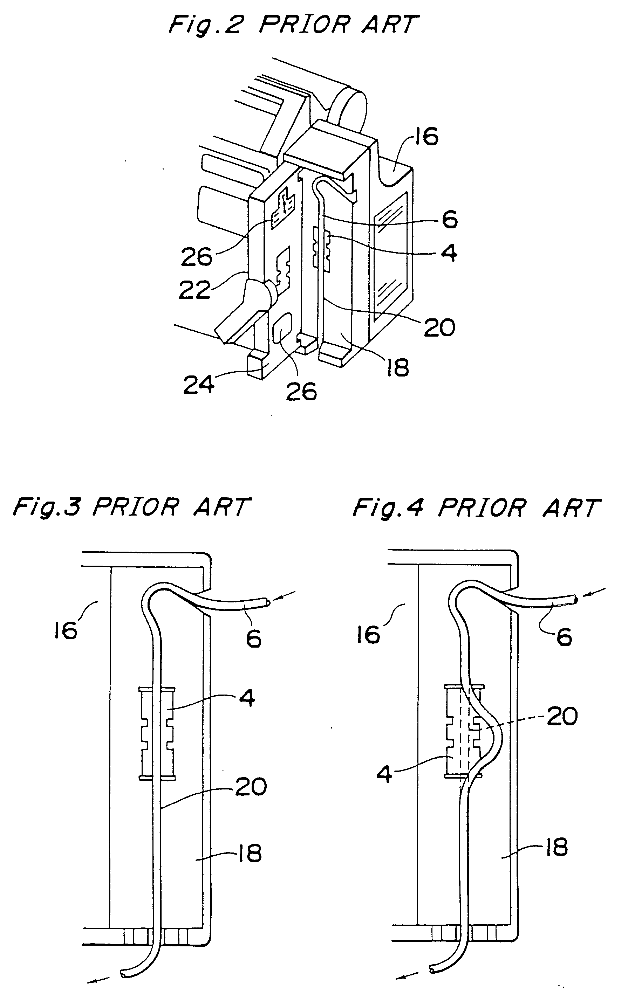

Fig. 2 depicts a solution housing 16 accommodating the solution bag 2. The tube 6 having one end connected to the solution bag 2 is led from the inside of the solution housing 16 to a tube receiving surface 18. The tube 6 is received in a tube path 20 formed on the tube receiving surface 18. The tube path 20 is a groove having an appropriate depth and an appropriate width to receive the tube 6. The solution actuator 4 is mounted on the tube receiving surface 18 and is in abutment with an intermediate portion of the tube 6. The solution actuator 4 presses or squeezes the solution 3 inside the tube 6 from the upstream side (the side of the solution bag 2) towards the downstream side (the side of the human body 8). To this end, the solution actuator 4 comprises a solution delivery pump, an eccentric cam secured to a rotary shaft of the solution delivery pump, and a plurality of fingers fixedly mounted on the outer circumference of the eccentric cam. The rotation of the eccentric cam along with the rotary shaft of the solution delivery pump enables the fingers to press the tube 6 downwards so that the solution 3 may flow towards the human body 8.

The solution housing 16 is provided with a door 22 hingedly connected thereto. The door 22 can move between its open position and its closed position where the door 22 closes the tube receiving surface 18 of the solution housing 16. The door 22 has a flat inner surface 24 on which are mounted a plurality of sensors 26 such as, for example, pressure sensors for detecting the solution conditions. When the door 22 is closed with respect to the tube receiving surface 18 with the tube 6 being placed in position in the tube path 20, as shown in Fig. 3, the tube receiving surface 18 and the inner surface 24 of the door 22 are in abutment with each other.

However, if the tube 6 is not appropriately received in the tube path 20, as shown in Fig. 4, i.e., if the former deviates off the latter, the solution actuator 4 does not operate normally and the tube 6 is subjected to deformation by the pressure of the door 22 against the tube 6. As a result, the rate of feed of the solution extremely reduces or no solution is fed to a patient. In such a case, the patient is unlikely to undergo solution treatment.

US-A-4,174,637 on which the preamble of claim 1 is based, discloses a pressure monitoring system with a pressure transducer which is mounted in an opening of a tube mounting means nearby the fluid line. The single pressure transducer is provided to monitor the internal system pressure of the fluid line, but is also capable of giving an alarm in case of improper insertion of the fluid line in the tube mounting means. It is a problem of this embodiment that the pressure transducer only monitors one point of the fluid line and that the rest of the fluid line is not under control. Therefore it could be that parts of the fluid line are improperly inserted in the tube mounting means, although the pressure transducer does not give an alarm.

### Summary of the invention

The present invention has been developed to overcome the above-described disadvantage.

It is accordingly an object of the present invention to provide an improved solution infusion system capable of detecting improper mounting of a solution tube.

When the improper mounting of the solution tube is detected, the system gives a warning for the rapid removal of a cause thereof so that a patient may undergo appropriate solution treatment.

In accomplishing the above and other objects, a solution infusion system according to the present invention comprises a solution housing having solution tube mounting means, a solution tube to be placed in a tube path formed on the solution tube mounting means, and pressure-sensitive means for detecting whether the solution tube is properly placed in the tube path. The pressure-sensitive means outputs a detection signal in association with the placement of the solution tube. The solution infusion system according to the present invention further comprises control means for controlling the amount of a solution to be fed through the solution tube. When the solution tube deviates off the tube path, the control means gives a warning in response to the detection signal outputted from the pressure-sensitive means.

The pressure-sensitive means comprises a pair of pressure sensors disposed on respective sides of the solution tube.

The solution tube mounting means comprises a first surface and a second surface formed on the solution housing. These two surfaces are in abutment with each other when the system is in operation. In this case, the tube path is formed on the first surface, and the pressure sensors are disposed on respective sides of the tube path and detect the pressure applied thereto by the first and second surfaces. When the pressure detected by at least one of the pressure sensors is less than a predetermined value, the control means determines that the solution tube deviates off the tube path.

The second surface presses the first surface along with the solution tube received in the tube path. In this case, the pressure applied to the first surface by the second surface at the time the solution tube deviates off the tube path is greater than the pressure applied at the time the former is placed in position in the latter. Based on this pressure, the pressure sensors output respective detection signals indicative of whether the solution tube is properly received in the tube path.

### BRIEF DESCRIPTION OF THE DRAWINGS

The above and other objects and features of the present invention will become more apparent from the following description of a preferred embodiment thereof with reference to the accompanying drawings, throughout which like parts are designated by like reference numerals, and wherein:
Fig. 1 is a block diagram of a conventional solution infusion system;
Fig. 2 is a fragmentary perspective view of a solution housing of the conventional solution infusion system;
Fig. 3 is a fragmentary side view of the solution housing with a door opened, indicating the case in which a solution tube is properly mounted;
Fig. 4 is a view similar to Fig. 3, but indicating the case in which the solution tube is improperly mounted;
Fig. 5 is a block diagram of a solution infusion system according to the present invention;
Fig. 6 is a fragmentary perspective view of a solution housing of the solution infusion system of Fig. 5;
Fig. 7 is a fragmentary side view of the solution housing with a door opened, indicating the case in which a solution tube is properly mounted;
Fig. 8 is a view similar to Fig. 7, but indicating the case in which the solution tube is improperly mounted;
Fig. 9 is a graph indicative of the resistance value of each pressure sensor with respect to the magnitude of the pressure applied thereto; and
Fig. 10 is a flow-chart indicative of the control operation carried out in the system of Fig. 5.

### DETAILED DESCRIPTION OF THE PREFERRED EMBODIMENTS

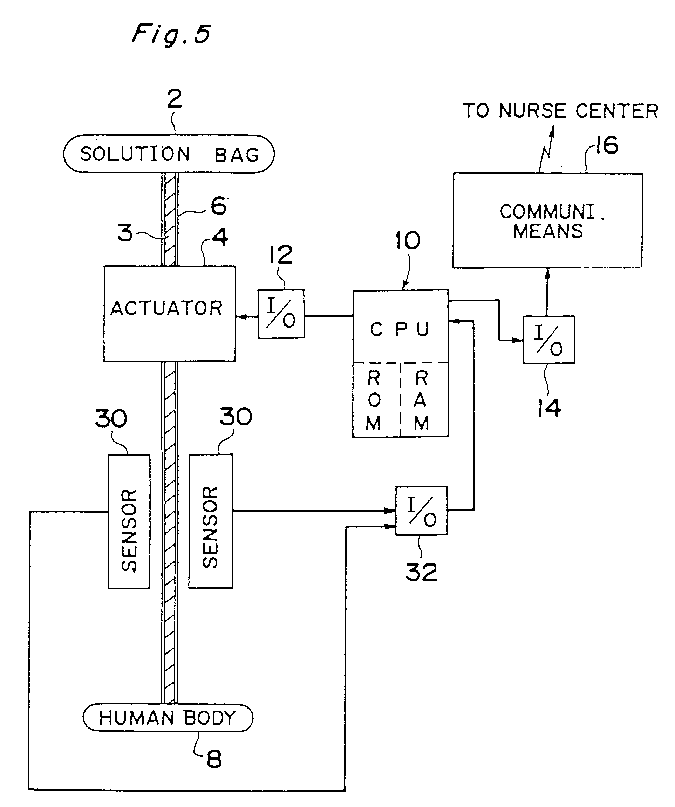

Referring now to the drawings, there is shown in Fig. 5 a solution infusion system embodying the present invention. The solution infusion system of Fig. 5 comprises a solution bag 2 containing a solution 3, a solution actuator 4 for effecting pumping action, and a solution tube 6 for introducing the solution 3 into a human body 8. The solution infusion system also comprises a control unit 10 provided with a microcomputer. The microcomputer comprises a CPU for executing a main control operation, a ROM in which is stored an infusion operation control program, and a RAM for temporarily storing data. The control unit 10 controls the solution actuator 4 via an I/O interface 12 in accordance with the infusion operation control program, thereby feeding an appropriate amount of solution to the human body 8.

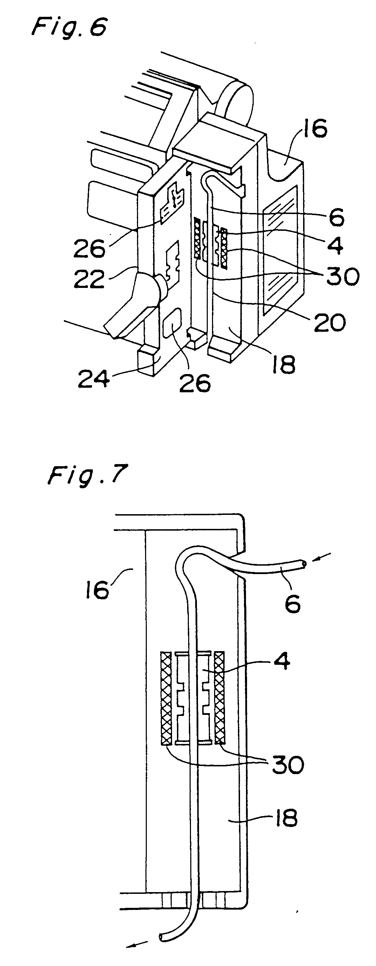

Fig. 6 depicts a solution housing 16 accommodating the solution bag 2. The tube 6 having one end connected to the solution bag 2 is led from the inside of the solution housing 16 to a tube receiving surface 18. The tube 6 is received in a tube path 20 formed on the tube receiving surface 18. The tube path 20 is a groove having an appropriate depth and an appropriate width to receive the tube 6. The solution actuator 4 is mounted on the tube receiving surface 18 and is in abutment with an intermediate portion of the tube 6. The solution actuator 4 presses or squeezes the solution 3 inside the tube 6 so that the solution 3 may flow towards the human body 8.

The solution housing 16 is provided with a door 22 hingedly connected thereto. The door 22 can move between its open position and its closed position where the door 22 closes the tube receiving surface 18 of the solution housing 16. The door 22 has a flat inner surface 24 on which are mounted a plurality of sensors 26 such as, for example, pressure sensors for detecting the solution conditions. When the door 22 is closed with respect to the tube receiving surface 18 with the tube 6 being placed in position in the tube path 20, the tube receiving surface 18 and the inner surface 24 of the door 22 are in abutment with each other.

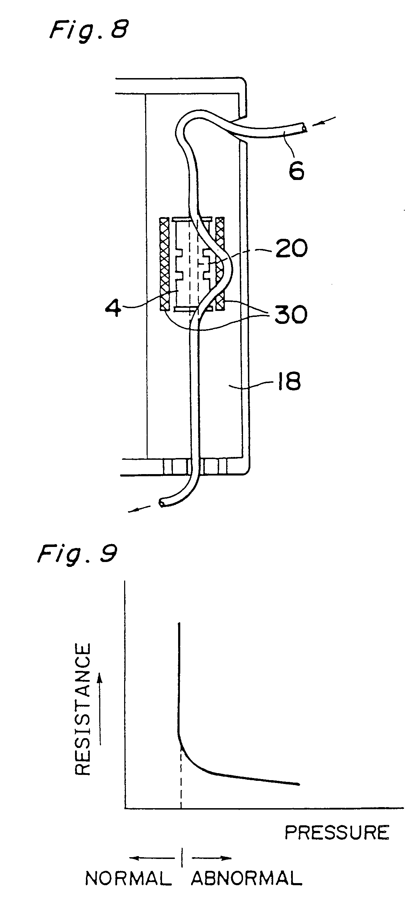

As shown in Figs. 5 through 7, the solution infusion system according to the present invention further comprises a pair of pressure sensors 30 disposed on the tube receiving surface 18 on respective sides of the solution actuator 4. Alternatively, the pressure sensors 30 may be disposed on the inner surface 24 of the door 22 so that these sensors 30 may be placed on respective sides of the solution actuator 4 when the door 22 is closed. The pressure sensors 30 may be extended beyond the solution actuator 4 along the tube path 20. The pressure sensors 30 detect whether the tube 6 is placed in position in the tube path 20 and are made of, for example, conductive rubber. If a pressure is applied to one or both of the pressure sensors 30 and causes deformation thereof, the resistance value thereof reduces and the pressure sensors 30 output detection signals corresponding to the reduction in resistance value. More specifically, when the tube 6 is placed in position in the tube path 20, as shown in Fig. 7, the tube receiving surface 18 and the inner surface 24 of the door 22 are in abutment with each other at a relatively low pressure. In this case, the pressure sensors 30 indicate respective high resistance values. In contrast, if the tube 6 is not appropriately received in the tube path 20, for example, if the tube 6 is placed on one of the pressure sensors 30, as shown in Fig. 8, the inner surface 24 of the door 22 applies a relatively large pressure to the tube receiving surface 18. As a result, associated one of the pressure sensors 30 indicates a low resistance value.

Fig. 9 is a graph indicative of the resistance value of each pressure sensor 30, which changes with the magnitude of the pressure applied thereto.

The control unit 10 also comprises a warning controller. When the tube 6 deviates off the tube path 20, the warning controller gives a warning in response to a detection signal or signals outputted from the pressure sensors 30 via an I/O interface 32. This warning is transmitted to, for example, a nurse center via an I/O interface 14 and any suitable communication means 16.

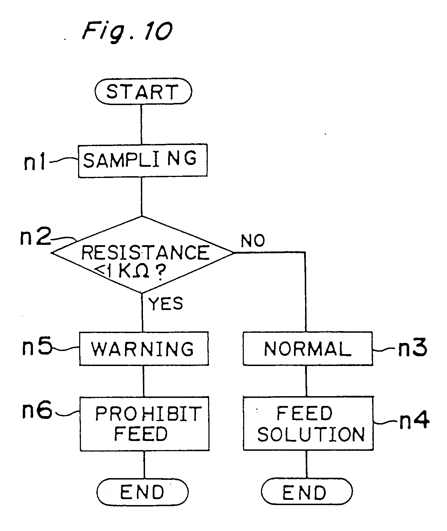

The control operation, which is mainly carried out by the control unit 10, is discussed hereinafter with reference to Figs. 7 through 10.

After the door 22 has been closed, the control operation starts. Step n1 establishes a sampling condition to check the solution conditions. Step n2 determines whether the resistance value of each pressure sensor 30 is less than a predetermined value, for example 1 kΩ. If the former exceeds the latter, it is judged that the tube 6 is placed in position in the tube path 20. In contrast, if the resistance value of at least one of the pressure sensors 30 is less than the predetermined value, it is judged that the tube 6 is improperly mounted on the tube receiving surface 18. When the resistance value of one of the pressure sensors 30 exceeds the predetermined value, step n3 determines that the tube 6 is properly received in the tube path 20. At step n4, the system allows the feed of the solution 3. In contrast, when the resistance value of each pressure sensor 30 is less than the predetermined value, step n5 determines that the tube 6 is improperly mounted on the tube receiving surface 18 and gives a warning. At step n6, the system prohibits the feed of the solution 3.

As is clear from the above, improper mounting of the solution tube 6 is detected by the pressure sensors 30, and a warning is given in response to a detection signal outputted from at least one of the pressure sensors 30 to urge a doctor or a nurse to remove a cause thereof. As a result, a patient can undergo appropriate solution treatment before it is too late.

Although the present invention has been fully described by way of examples with reference to the accompanying drawings, it is to be noted here that various changes and modifications will be apparent to those skilled in the art. Therefore, unless such changes and modifications otherwise depart from the scope of the present invention as defined in the appended claims, they should be construed as being included therein.

## Claims

1. A solution infusion system for infusing a solution (3) into a human body (8), said solution infusion system comprising:
a solution housing (16) having solution tube mounting means (18, 24);
a solution tube (6) to be placed in a tube path (20) formed on said solution tube mounting means (18, 24);
pressure-sensitive means (30) for detecting whether said solution tube (6) is properly placed in said tube path (20), said pressure-sensitive means (30) outputting a detection signal in association with placement of said solution tube (6); and
control means (10) for controlling an amount of a solution (3) to be fed through said solution tube (6), said control means (10) giving a warning in response to said detection signal outputted from said pressure-sensitive means (30) when said solution tube (6) deviates off said tube path (20), characterized in that said
pressure-sensitive means (30) comprises a pair of pressure sensors (30) disposed on respective sides of said solution tube (6); and that
said solution tube mounting means (18, 24) comprises a first surface (18) and a second surface (24) formed on said solution housing (16), said first surface (18) and said second surface (24) being in abutment with each other when the system is in operation, wherein said tube path (20) is formed on said first surface (18), and wherein said pressure sensors (30) are disposed on respective sides of said tube path (20) and detect a pressure applied thereto by said first and second surfaces (18, 24).

2. The system according to claim 1, wherein, when the pressure detected by at least one of said pressure sensors (30) is less than a predetermined value, said control means (10) determines that said solution tube (6) deviates off said tube path (20).

## Patentansprüche

1. Flüssigkeitsinfusionssystem zur Infusion einer Flüssigkeitslösung (3) in einen menschlichen Körper (8), mit:
einem Flüssigkeitsbehälter (16) mit einer Flüssigkeitsschlauch-Befestigungseinrichtung (18, 24);
einem in einen durch die Flüssigkeitsschlauch-Befestigungseinrichtung (18, 24) gebildeten Schlauchweg (20) einzusetzendenden Flüssigkeitsschlauch (6);
einer druckempfindlichen Einrichtung (30) zum Bestimmen, ob der Flüssigkeitsschlauch (6) korrekt in den Schlauchweg (20) eingesetzt ist, wobei die druckempfindliche Einrichtung (30) ein Erkennungssignal beim Einsetzen des Flüssigkeitsschlauchs (6) ausgibt; und mit
einer Steuerungseinrichtung (10) zum Steuern einer durch den Flüssigkeitsschlauch (6) zuzuführenden Menge von Flüssigkeitslösung (3), wobei die Steuerungseinrichtung (10) eine Warnung in Abhängigkeit von dem durch die druckempfindliche Einrichtung (30) ausgegebenen Erkennungssignal abgibt, wenn der Flüssigkeitsschlauch (6) von dem Schlauchweg (20) abweicht, **dadurch gekennzeichnet**, daß die druckempfindliche Einrichtung (30) ein Paar von an den jeweiligen Seiten des Flüssigkeitsschlauchs (6) angeordneten Drucksensoren (30) aufweist; und daß die Flüssigkeitsschlauch-Befestigungseinrichtung (18, 24) eine erste Oberfläche (18) und eine zweite Oberfläche (24), die jeweils an dem Flüssigkeitsbehälter (16) ausgebildet sind, aufweist, wobei die erste Oberfläche (18) und die zweite Oberfläche (24) einander berühren, wenn das System in Betrieb ist, und der Schlauchweg (20) auf der ersten Oberfläche (18) gebildet ist, und die Drucksensoren (30) auf den jeweiligen Seiten des Schlauchwegs (20) angeordnet sind und einen durch die erste (18) und zweite (24) Oberfläche darauf aufgebrachten Druck bestimmen.

2. System nach Anspruch 1, **dadurch gekennzeichnet**, daß, wenn der durch mindestens einen der Drucksensoren (30) bestimmte Druck geringer ist als ein vorbestimmter Wert, die Steuerungseinrichtung (10) bestimmt, daß der Flüssigkeitsschlauch (6) von dem Schlauchweg (20) abweicht.

## Revendications

1. Système de perfusion de solution liquide pour injecter une solution liquide (3) dans un corps humain (8), ledit système de perfusion de solution liquide comprenant:
un logement de solution (16) ayant des moyens de montage de tube de solution (18, 24);
un tube de solution (6) à mettre en place dans un chemin de tube (20) formé sur lesdits moyens de montage de tube de solution (18, 24);
des moyens sensibles à la pression (30) pour détecter si oui ou non ledit tube de solution (6) est correctement mis en place dans ledit chemin de tube (20), lesdits moyens sensibles à la pression (30) délivrant un signal de détection en association avec la mise en place dudit tube de solution (6); et
des moyens de commande (10) pour commander une quantité de solution (3) à fournir à travers ledit tube de solution (6), lesdits moyens de commande (10) délivrant un signal d'avertissement en réponse audit signal de détection délivré par lesdits moyens sensibles à la pression (30) lorsque ledit tube de solution (6) s'écarte dudit chemin de tube (20), caractérisé en ce que
lesdits moyens sensibles à la pression (30) comprennent une paire de détecteurs de pression (30) disposés sur des côtés respectifs dudit tube de solution (6); et en ce que
lesdits moyens de montage de tube de solution (18, 24) comprennent une première surface (18) et une seconde surface (24) formées sur ledit logement de solution (16), ladite première surface (18) et ladite seconde surface (24) étant en contact l'une avec l'autre lorsque le système est en fonctionnement, dans lequel ledit chemin de tube (20) est formé sur ladite première surface (18), et dans lequel lesdits détecteurs de pression (30) sont disposés sur des côtés respectifs dudit chemin de tube (20) et détectent une pression qui leur est appliquée par lesdites première et seconde surfaces (18, 24).

2. Système selon la revendication 1, dans lequel, lorsque la pression détectée par au moins l'un desdits détecteurs de pression (30) est inférieure à une valeur prédéterminée, lesdits moyens de commande (10) détectent que ledit tube de solution (6) s'écarte dudit chemin de tube (20).
